(19) Europäisches Patentamt / European Patent Office / Office européen des brevets

(11) **EP 3 662 972 B1**

(12) **FASCICULE DE BREVET EUROPEEN**

(45) Date de publication et mention
de la délivrance du brevet:
**25.08.2021 Bulletin 2021/34**

(51) Int Cl.:
***A61N 1/375*** *(2006.01)*     *A61N 1/36* *(2006.01)*

(21) Numéro de dépôt: **19212796.7**

(22) Date de dépôt: **02.12.2019**

(54) **DISPOSITIF MÉDICAL IMPLANTABLE À ARCHITECTURE AMÉLIORÉE**

IMPLANTIERBARES MEDIZINISCHES GERÄT MIT VERBESSERTER ARCHITEKTUR

IMPLANTABLE MEDICAL DEVICE WITH IMPROVED ARCHITECTURE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **07.12.2018 FR 1872524**

(43) Date de publication de la demande:
**10.06.2020 Bulletin 2020/24**

(73) Titulaire: **Commissariat à l'Énergie Atomique
et aux Énergies Alternatives
75015 Paris (FR)**

(72) Inventeurs:
• **SAUTER-STARACE, Fabien
38054 Grenoble Cedex 9 (FR)**
• **SIEGEL, Alice
38054 Grenoble Cedex 9 (FR)**

(74) Mandataire: **INNOV-GROUP
310, avenue Berthelot
69372 Lyon Cedex 08 (FR)**

(56) Documents cités:
WO-A2-2006/081361     CN-A- 106 512 213
FR-A1- 2 898 462

EP 3 662 972 B1

## Description

### Domaine technique de l'invention

La présente invention se rapporte à un dispositif médical implantable. Le dispositif médical implantable de l'invention présente la particularité d'être très résistant aux chocs.

### Etat de la technique

Dans le domaine des dispositifs médicaux implantables actifs (appelés DMIA) en particulier les implants intracrâniens tels que les interfaces cerveaux machine, les implants cochléaires et les neurostimulateurs, il est nécessaire de répondre à certaines contraintes :

- Le boîtier du dispositif doit être hermétique pour protéger le patient des éventuels risques biologiques involontairement causés par le dispositif DMIA et pour éviter d'endommager l'électronique interne ;
- Le dispositif doit être aussi capable de dissiper la chaleur générée par le fonctionnement de l'électronique interne ;
- Le dispositif doit être capable de résister aux chocs. En cas de chocs, ces dispositifs doivent conserver au maximum leur intégrité et ne doivent pas léser le cerveau ou la boîte crânienne ;

La demande de brevet n°WO2006/081361A2 est relative aux implants cochléaires. Elle décrit un dispositif qui comporte un conteneur hermétique dans lequel est placée l'électronique du dispositif et un couvercle venant être apposé sur le conteneur hermétique et définissant un espace interne non hermétique avec ledit conteneur. La demande de brevet précise qu'un matériau incluant de la silicone peut être injecté dans cet espace interne, en vue notamment de renforcer la résistance aux chocs du dispositif.

Cependant, l'architecture proposée dans ce document n'est pas satisfaisante car elle ne permet pas de répondre à toutes les contraintes évoquées ci-dessus. En effet, elle ne permet pas une bonne dissipation thermique puisque l'électronique est isolée dans le conteneur hermétique, aucune solution n'étant prévue pour dissiper la chaleur générée en fonctionnement. De plus, elle ne permet pas de répondre aux différents types de chocs qui peuvent survenir, ceux-ci pouvant causer des déformations élastiques ou des déformations plastiques du dispositif.

Le but de l'invention est donc de proposer un dispositif médical implantable qui permet de palier les inconvénients de l'état de la technique et qui puisse répondre aux différentes contraintes de fonctionnement listées ci-dessus.

### Exposé de l'invention

Ce but est atteint par un dispositif médical implantable comprenant :

- Un boitier hermétique définissant un espace interne,
- Au moins une carte électronique logée dans ledit espace interne du boîtier, ladite carte supportant des composants électroniques,
- Un module de dissipation thermique et d'absorption des chocs, logé dans l'espace interne du boîtier et agencé entre ladite carte électronique et une paroi interne du boîtier,

Ledit module de dissipation thermique et d'absorption des chocs comportant :

- Une première couche de pâte thermique placée dans l'espace interne du boîtier et déposée sur ladite carte électronique,
- Une deuxième couche métallique à haute conductivité thermique déposée sur ladite première couche,
- Une troisième couche de pâte thermique déposée sur ladite deuxième couche et agencée pour venir en appui contre ladite paroi interne du boîtier, ladite troisième couche comportant une structure présentant plusieurs cavités réparties sur toute la zone de contact de la troisième couche avec la paroi interne du boîtier.

Selon une réalisation particulière, la structure de la troisième couche est réalisée sous la forme de plusieurs cavités concentriques.

Selon une autre réalisation particulière, la structure de la troisième couche comporte plusieurs cavités rectilignes parallèles formant des tranchées et une structure en peigne présentant plusieurs dents de hauteur et de largeur déterminées.

Selon une autre réalisation particulière, la structure de la troisième couche comporte une spirale.

Selon une particularité du dispositif, chaque cavité de la structure est traversante.

Selon une autre particularité, la deuxième couche est formée d'un métal de forte conductivité thermique tel que le cuivre, l'or ou l'aluminium.

Selon une autre particularité, la pâte thermique de la première couche et de la troisième couche présente une conductivité thermique comprise entre 1 et 12 W/m/K.

Selon une autre particularité, la première couche et la troisième couche comporte de la silicone chargée.

Selon une autre particularité, le module de dissipation thermique et d'absorption des chocs comporte une ou plusieurs couches supplémentaires faites de graphite pyrolytique, déposées sous la deuxième couche et entre la troisième couche et la paroi interne du boitier

L'invention concerne également un procédé de fabrication d'un dispositif médical implantable tel que défini ci-dessus, la structure de la troisième couche étant réalisée en appliquant des emporte-pièces sur une couche de pâte thermique déposée sur un substrat, par moulage par compression ou par moulage par injection dans un moule soluble chimiquement ou liquéfiable thermiquement.

## Brève description des figures

D'autres caractéristiques et avantages vont apparaître dans la description détaillée qui suit faite en regard des dessins annexés dans lesquels :

- La figure 1 représente, vue en coupe et de manière schématique, l'architecture du dispositif médical implantable conforme à l'invention ;
- Les figures 2A à 2D représentent trois exemples de structuration possible de la troisième couche employée dans le dispositif de l'invention ;
- Les figures 3 à 5 montrent respectivement trois procédés de réalisation de la troisième couche structurée employée dans le dispositif de l'invention ;
- Les figures 6A à 6C et 7A à 7C illustrent le principe d'absorption des chocs permis grâce au dispositif de l'invention ;

## Description détaillée d'au moins un mode de réalisation

Dans la suite de la description et sur les dessins, les termes "haut", "bas", "supérieur", "inférieur", "au-dessus", "au-dessous" ou termes équivalents sont à comprendre en tenant compte d'un axe principal (X) tracé verticalement.

La solution de l'invention s'applique principalement aux dispositifs médicaux implantables actifs (DMIA), en particulier ceux dont la consommation énergétique est élevée. Les stimulateurs implantables de type "pacemaker" ont fait l'objet de nombreuses optimisations et consomment très peu d'énergie électrique (quelques $\mu$W) et dissipent donc très peu de chaleur. En revanche, les systèmes d'enregistrement de type BCI ("Brain Computer Interface") peuvent consommer jusqu'à 350mW (voir publication référencée "Mestais, C. S., Charvet, G., Sauter-Starace, F., Foerster, M., Ratel, D., & Benabid, A. L. (2015). WIMAGINE: wireless 64-channel ECoG recording implant for long term clinical applications. IEEE transactions on neural systems and rehabilitation engineering, 23(1), 10-21"). Pour ces dispositifs, la problématique d'une extraction efficace et homogène de l'énergie devient donc critique.

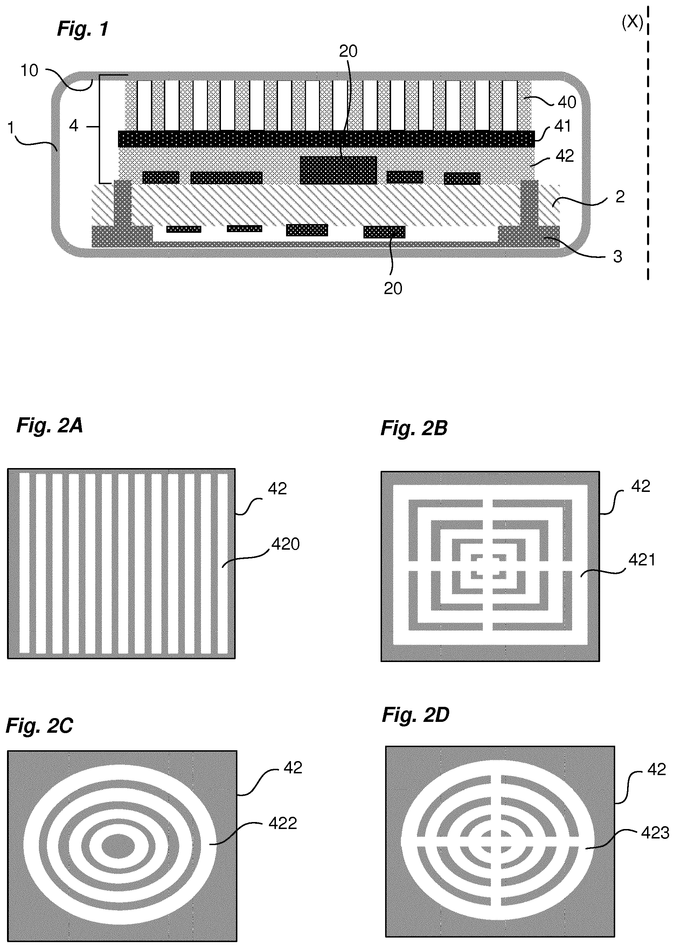

En référence à la figure 1, un dispositif médical implantable conforme à l'invention, comporte un boîtier 1 pouvant être réalisé en deux parties assemblées l'une sur l'autre, une première partie, dite inférieure, formant un conteneur, et une deuxième partie, dite supérieure, formant un couvercle venant se positionner sur le conteneur. Lorsque les deux parties sont assemblées, le boîtier 1 est fermé de manière hermétique et définit un espace interne isolé de l'extérieur. L'herméticité entre les deux parties peut être réalisée par tous moyens connus.

Pour fonctionner, le dispositif comporte une carte électronique 2. La carte électronique 2 comporte deux faces, chacune de ses faces pouvant supporter un ou plusieurs composants électroniques 20.

Le dispositif peut comporter un châssis 3 logé dans le fond d'une des deux parties du boîtier 1, sur lequel est fixée la carte électronique 2. Sur la figure 1, le châssis 3 est positionné dans le fond du conteneur. La carte 2 est montée par sa face inférieure sur le châssis et peut être fixé sur le châssis 3 par tous moyens appropriés. Le châssis 3 peut être une pièce indépendante fixée dans le fond du conteneur du boîtier, par exemple par des vis, ou directement moulé avec le conteneur du boîtier de manière à présenter des organes de support pour la carte électronique 2.

Dans l'espace interne du boitier, le dispositif médical implantable comporte également un module 4 de dissipation thermique et d'absorption des chocs.

Ce module 4 comporte un ensemble de plusieurs couches 40, 41, 42 superposées. L'ensemble de couches superposées comporte une face inférieure déposée sur la face supérieure de la carte électronique 2, à l'opposé de sa face inférieure de fixation sur le châssis 3 et une face supérieure contre laquelle la paroi interne 10 de la partie supérieure du boîtier 1 vient en appui, permettant de caler le dispositif de dissipation thermique et d'absorption des chocs dans le boîtier et de servir d'absorbeur de chocs lorsque le boîtier reçoit des chocs sur sa face supérieure.

Le module de dissipation thermique et d'absorption des chocs comporte :

- Une première couche 40 formée d'une pâte thermique et isolante électriquement déposées sur la face supérieure de la carte électronique. Si des composants électroniques sont montés sur cette face, la pâte vient les enrober lors de son dépôt ;
- Une deuxième couche 41 métallique à haute conductivité thermique, déposée sur la face supérieure de la première couche ;
- Une troisième couche 42 qui est également formée d'une pâte thermique et qui est déposée sur la face supérieure de la deuxième couche, sa face supérieure venant en appui contre la paroi interne de la partie supérieure du boîtier.

La première couche 40 et la troisième couche 42 peuvent être réalisées dans une même pâte thermique. Pour la troisième couche 42, ce matériau est choisi pour assurer une excellente conductivité thermique et présenter une composition suffisamment déformable pour absorber les chocs.

De manière non limitative, le matériau choisi pour réaliser la pâte thermique peut être de la silicone chargée. Il peut ainsi contenir de l'oxyde de zinc, en plus de la silicone qui est utilisée comme agent liant permettant à la conductivité thermique de passer de 0.2W/m/K à des valeurs entre 3 et 5W/m/K. Si l'isolation électrique des composants est assurée par un vernis de type acrylique ou une couche d'époxy, d'autres pâtes thermiques peuvent également être citées avec des conductivités thermiques allant jusqu'à 12W/m/K. Le matériau dénommé "Prolimatech PK1" (marque déposée) est composé de 60 à 85 % d'aluminium, 15-25 % d'oxyde de zinc, 12-20 % d'huile de silicone et enfin d'un agent antioxydant. Certaines pâtes conductrices thermiques, comme celle qui est dénommé "Arctic Silver 5" (marque déposée) contiennent en plus des particules d'argent. D'autres font appel au graphite comme la pâte référencée "WLPG 10" (marque déposée) de Fischer Elektronik, celle-ci n'employant pas de silicone et affichant une excellente conductivité thermique (10,5 W/m·K). Enfin, il est également connu d'y ajouter des nanoparticules de carbone.

Selon une particularité, la pâte thermique employée présente une conductivité thermique comprise entre 1 et 12 W/m/K.

La deuxième couche 41 employée est avantageusement une couche métallique afin d'intégrer un matériau à forte conductivité thermique dans le module 4 de dissipation thermique et d'absorption des chocs. Il peut s'agir d'une plaque de cuivre, d'or ou d'aluminium. La deuxième couche peut présenter une épaisseur comprise entre 0.1 et 0.6mm. Comme cette deuxième couche s'avère être relativement rigide, la troisième couche réalisée dans un matériau plus souple et déformable est nécessaire pour assurer une fonction d'absorption des chocs et proposer un dispositif implantable qui présente un niveau de déformabilité supérieur à un dispositif qui ne comporterait qu'une seule couche métallique disposée entre la carte et le couvercle.

De manière optionnelle, pour améliorer le transfert thermique, le module 4 de dissipation thermique et d'absorption des chocs peut comporter une ou plusieurs couches supplémentaires faites de graphite pyrolytique, déposées sous la couche de cuivre et éventuellement entre la troisième couche 42 et la paroi interne 10 du couvercle du boitier.

Selon un aspect particulier de l'invention, la troisième couche 42, réalisée en pâte thermique, est dite structurée. Par "structurée", on entend qu'elle comporte plusieurs cavités afin d'améliorer sa déformabilité et ainsi mieux protéger l'ensemble électronique des chocs. La structure est ainsi réalisée pour alterner des parties pleines et des parties creuses.

Les cavités peuvent être traversantes (c'est-à-dire réalisées à travers toute l'épaisseur de la couche) ou non. La structure de la troisième couche 42 peut comporter un mélange de plusieurs cavités, certaines étant traversantes et d'autres non.

Les figures 2A à 2D montrent plusieurs exemples de structures possibles au niveau de la troisième couche.

Sur la figure 2A, la structure est dite en peigne. Elle comporte ainsi plusieurs tranchées 420 traversantes rectilignes parallèles, formant ainsi plusieurs traverses ou dents parallèles.

Sur la figure 2B, la structure comporte plusieurs cavités 421 traversantes en rectangle, disposées de manière concentrique. Deux cavités suivant deux tranchées rectilignes perpendiculaires entre elles peuvent également être ajoutées.

Sur la figure 2C, la structure comporte plusieurs cavités traversantes circulaires 422 ou elliptiques, disposées de manière concentrique.

Sur la figure 2D, la structure comporte plusieurs cavités traversantes circulaires 423, disposées de manière concentrique, deux tranchées rectilignes et perpendiculaires entre elles étant ajoutées.

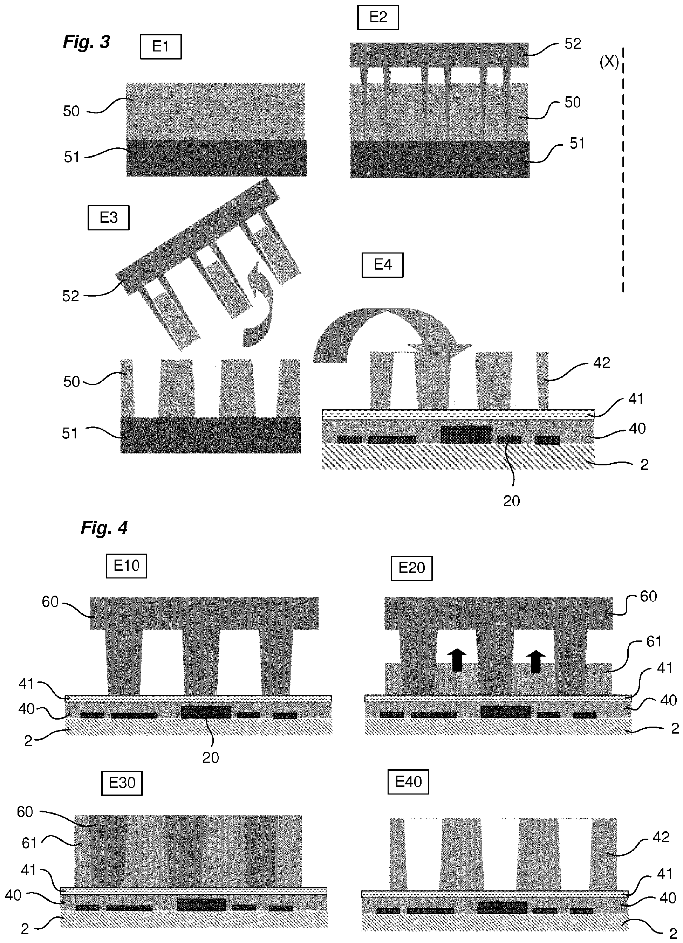

Plusieurs méthodes peuvent être envisagées pour la réalisation de la structure dans la troisième couche. Les figures 3 à 5 montrent plusieurs méthodes distinctes pour réaliser une structure en peigne, telle que celle de la figure 2A, dans une pâte thermique.

La figure 3 montre une première méthode de réalisation. Cette première méthode comporte les étapes suivantes :

E1 : Une couche 50 de pâte conductrice thermique à structurer est déposée de manière homogène sur un substrat 51. L'épaisseur de la couche 50 peut être est homogénéisée au moyen d'une racle ou d'une plaque venant en butée sur une cale d'épaisseur.

E2 : La structure est réalisée en employant un outil 52 doté avantageusement de plusieurs emporte-pièces. Les emporte-pièces ont avantageusement une forme adaptée à la structure finale à réaliser, par exemple en forme de peigne, d'anneau, de rectangle... L'outil est appliqué à travers la couche de matériau de manière à ce que les emporte-pièces traversent ladite couche.

E3 : Le retrait de l'outil 52 permet d'enlever la matière et d'obtenir la structure souhaitée.

E4 : La couche 50 structurée obtenue peut ensuite être rapportée sur la deuxième couche 41 du module de dissipation thermique et d'absorption des chocs, pour devenir sa troisième couche 42.

La figure 4 montre une deuxième méthode de réalisation de la couche structurée. Cette solution peut être mise en oeuvre directement sur la deuxième couche 41 du module 4 de dissipation thermique et d'absorption des chocs ou sur un substrat séparé.

E10 On utilise une structure négative soluble chimiquement ou préférentiellement liquéfiable en température. Cette structure négative peut être un moule 60 thermosensible en cire ou paraffine (température de transition solide/liquide proche de 45°C), qui comprend une base et des structures en relief.

E20 : Une fois le moule 60 appliqué sur la deuxième couche 41, on injecte une quantité déterminée de pâte thermique 61.

E30 et E40 : On chauffe l'ensemble jusqu'à dissolution partielle puis complète du moule 60 employé. Il ne reste alors que la couche structurée 42 souhaitée, suite à la disparition du moule.

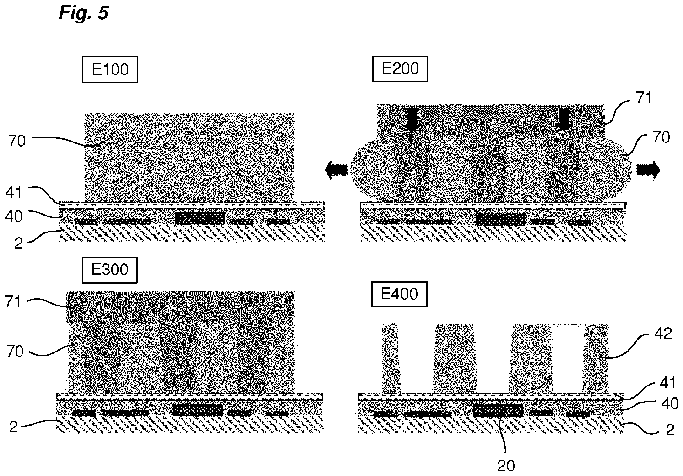

La figure 5 montre une dernière méthode de génération de la structure dans la troisième couche du module.

E100 : Une couche 70 de pâte thermique est déposée directement, avec une épaisseur homogène, sur la deuxième couche 41 du module 4.

E200 : On applique un moule 70 par compression contre la couche 70 jusqu'à la traverser. Le moule 70 est d'une forme adaptée à la structure à obtenir.

E300 : Le surplus de matière dégagée par la compression est retiré.

E400 : Le moule 70 est retiré afin d'obtenir la troisième couche 42 avec la structure choisie.

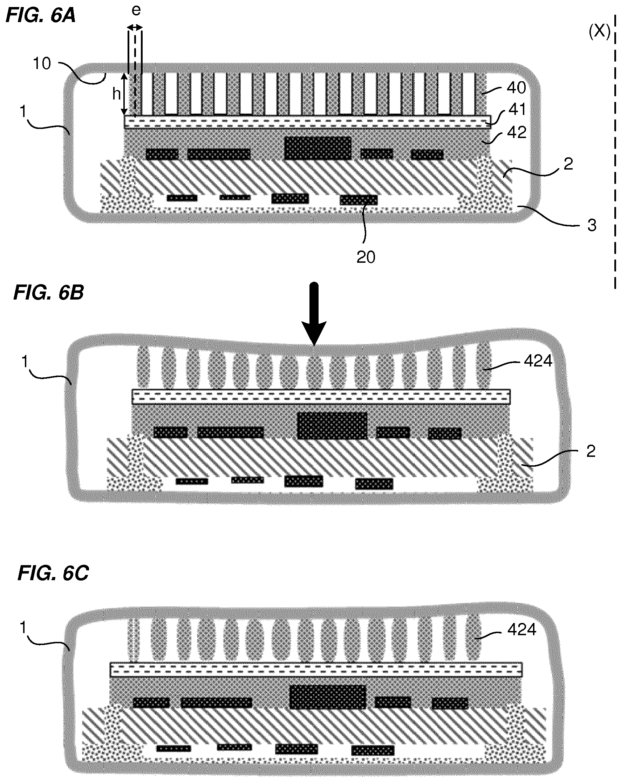

Les figures 6A à 6C représentent le dispositif conforme à l'invention soumis à une déformation allant au-delà de sa limite d'élasticité et permet de visualiser le principe d'absorption mécanique permis par le dispositif. Sur ces figures, la structure a une forme conforme à celle de la figure 2A, en peigne avec plusieurs traverses en parallèle.

Lorsque le boitier 1 est soumis à une contrainte mécanique celui-ci se déforme d'abord dans le domaine élastique (déformation réversible) puis dans le domaine plastique (déformation irréversible). L'enjeu de l'invention est ainsi d'absorber une partie de l'énergie de déformation pour éviter d'endommager l'électronique dans le boitier et de compromettre l'herméticité du dispositif.

Sur la figure 6A, un choc mécanique est appliqué contre la face supérieure du couvercle du boîtier 1 (suivant la flèche verticale).

Sur la figure 6B, le choc mécanique engendre une déformation du boîtier 1 et donc une compression des traverses 424 de la structure de la troisième couche 42.

Sur la figure 6C, après le choc mécanique, le boîtier 1 revient vers sa forme initiale s'il n'y a pas eu de déformation plastique. Le module 4 de dissipation thermique et d'absorption des chocs, notamment sa troisième couche 42 structurée, aura permis d'absorber une partie de l'énergie et de limiter la déformation du boîtier et d'éviter que celui-ci ne vienne endommager la partie électronique du dispositif.

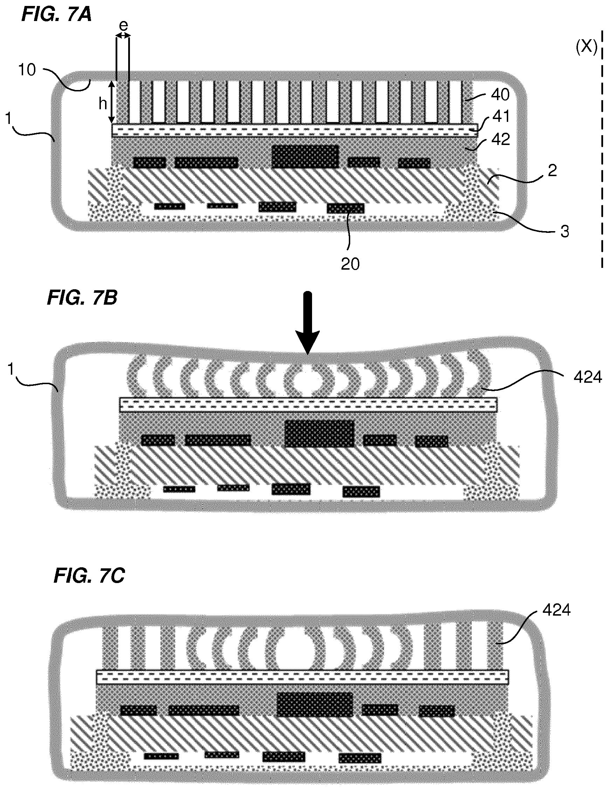

Sur les figures 7A à 7C, le principe est le même que précédemment, à la différence près que la structure de la troisième couche 42 ne se déforme pas en compression mais en flambement. Sur la figure 7B, on peut ainsi voir le flambement subi par les traverses 424 de la structure 42.

Chaque dent du peigne de la structure de la troisième couche, d'une hauteur h et d'une largeur e, est soumise à une contrainte dynamique de compression. Dans un régime stationnaire, la déformation de la dent est homogène et symétrique par rapport à la fibre neutre. Si l'élongation (h/e) est très élevée (>10), un flambement peut survenir au niveau de la dent, celle-ci se déforme alors principalement d'un seul côté. Pendant la phase de post-effondrement, une grande quantité d'énergie est dissipée par déformation plastique.

Cette charge critique au flambage F dépend du module de Young du matériau, des conditions de fixation des extrémités qui définissent la longueur de flambage ($l_k$ = h pour une poutre rotulée à ses deux extrémités) et du moment quadratique I de la poutre (et donc du coefficient e et de la profondeur p du peigne), selon la relation :

$$F = \frac{\pi^2 EI}{l_k^{\,2}}$$

Si cette dernière relation est appliquée à une seule dent, on trouve, pour un module de Young de 1MPa, une hauteur de 3mm, une largeur de peigne de 0.3mm et une profondeur de 10mm, une $F_{critique}$=0.025N.

Cette force est plus grande d'un facteur n, où n est le nombre de dents qui est également dépendant de la résolution du mode de réalisation.

Pour quantifier l'absorption d'énergie de la pâte thermique structurée et l'influence des facteurs de forme h, p et e, des simulations ont été mises en oeuvre.

Un premier type de simulation a été établi pour comparer le comportement de la pâte thermique non structurée à celui de la pâte thermique structurée.

La pâte thermique non structurée a été considérée comme un hexaèdre de dimensions : Profondeur 10 x largeur 4.8 x hauteur 3 mm$^3$.

Pour une pâte thermique structurée en peigne, les dents sont d'épaisseur e = h/10 = 0.3mm (h = 3 mm et p = 10 mm). Ce peigne est constitué de 8 dents identiques.

Ces deux structures sont soumises à une énergie d'impact de 75mJ (bloc de masse 6g impactant la pâte thermique à une vitesse de 5m/s).

Les résultats montrent que la pâte thermique structurée absorbe 50% de l'énergie d'impact, tandis que la pâte thermique non structurée n'en absorbe que 27%.

Les résultats indiquent ainsi qu'une structuration de la pâte thermique au niveau de la troisième couche 42 peut en effet lui conférer des propriétés améliorées d'absorption d'énergie.

On comprend de ce qui précède que le dispositif conforme à l'invention présente de nombreux avantages, parmi lesquels :

- Il permet de garantir une bonne dissipation thermique ;
- Il garantit de protéger l'électronique interne contre les chocs ponctuels et même contre les déformations plastiques irréversibles ;
- Il est d'une réalisation simple et utilise des matériaux classiques ;

## Revendications

1. Dispositif médical implantable comprenant :

   - Un boitier (1) hermétique définissant un espace interne,
   - Au moins une carte électronique (2) logée dans ledit espace interne du boîtier, ladite carte supportant des composants électroniques,
   - Un module (4) de dissipation thermique et d'absorption des chocs, logé dans l'espace interne du boîtier et agencé entre ladite carte électronique (2) et une paroi interne (10) du boîtier, et

   **caractérisé en ce que** ledit module de dissipation thermique et d'absorption des chocs comporte :

   - Une première couche (40) de pâte thermique placée dans l'espace interne du boîtier et déposée sur ladite carte électronique (2),
   - Une deuxième couche (41) métallique à haute conductivité thermique déposée sur ladite première couche (40),
   - Une troisième couche (42) de pâte thermique déposée sur ladite deuxième couche (41) et agencée pour venir en appui contre ladite paroi interne (10) du boîtier, ladite troisième couche (42) comportant une structure présentant plusieurs cavités réparties sur toute la zone de contact de la troisième couche avec la paroi interne du boîtier.

2. Dispositif selon la revendication 1, **caractérisé en ce que** la structure de la troisième couche (42) est réalisée sous la forme de plusieurs cavités (422) concentriques.

3. Dispositif selon la revendication 1, **caractérisé en ce que** la structure de la troisième couche (42) comporte plusieurs cavités (420) rectilignes parallèles formant des tranchées et une structure en peigne présentant plusieurs dents de hauteur et de largeur déterminées.

4. Dispositif selon la revendication 1, **caractérisé en ce que** la structure de la troisième couche (42) comporte une spirale.

5. Dispositif selon l'une des revendications 1 à 4, **caractérisé en ce que** chaque cavité de la structure est traversante.

6. Dispositif selon l'une des revendications 1 à 5, **caractérisé en ce que** la deuxième couche (41) est formée d'un métal de forte conductivité thermique tel que le cuivre, l'or ou l'aluminium.

7. Dispositif selon l'une des revendication 1 à 6, **caractérisé en ce que** la pâte thermique de la première couche (40) et de la troisième couche (42) présente une conductivité thermique comprise entre 1 et 12 W/m/K.

8. Dispositif selon l'une des revendications 1 à 7, **caractérisé en ce que** la première couche (40) et la troisième couche (42) comporte de la silicone chargée.

9. Dispositif selon l'une des revendications 1 à 8, **caractérisé en ce que** le module (4) de dissipation thermique et d'absorption des chocs comporte une ou plusieurs couches supplémentaires faites de graphite pyrolytique, déposées sous la deuxième couche (41) et entre la troisième couche (42) et la paroi interne (10) du boitier

10. Procédé de fabrication d'un dispositif médical implantable tel que défini dans l'une des revendications 1 à 9, **caractérisé en ce que** la structure de la troisième couche (42) est réalisée en appliquant des emporte-pièces sur une couche (50) de pâte thermique déposée sur un substrat (51), par moulage par compression ou par moulage par injection dans un moule (60) soluble chimiquement ou liquéfiable thermiquement.

## Patentansprüche

1. Implantierbares medizinisches Gerät, umfassend:

   - ein hermetisch dichtes Gehäuse (1), das einen Innenraum definiert,
   - mindestens eine Leiterplatte (2), die in dem Innenraum des Gehäuses aufgenommen ist, wobei die Leiterplatte elektronische Bauelemente trägt,
   - ein Wärmeableitungs- und Stoßabsorptionsmodul (4), das in dem Innenraum des Gehäuses aufgenommen ist und zwischen der Leiterplatte (2) und einer Innenwand (10) des Gehäuses angeordnet ist, **dadurch gekennzeichnet, dass** das Wärmeableitungs- und Stoßabsorptionsmodul beinhaltet:
   - eine erste Schicht (40) aus Wärmeleitpaste, die in dem Innenraum des Gehäuses platziert ist und auf die Leiterplatte (2) aufgebracht ist,
   - eine zweite Metallschicht (41) mit hoher Wärmeleitfähigkeit, die auf die erste Schicht (40) aufgebracht ist,
   - eine dritte Schicht (42) aus Wärmeleitpaste, die auf die zweite Schicht (41) aufgebracht ist und so angeordnet ist, dass sie an der Innenwand (10) des Gehäuses anliegt, wobei die dritte Schicht (42) eine Struktur beinhaltet, die mehrere Kavitäten aufweist, die über den gesamten Kontaktbereich der dritten Schicht mit der Innenwand des Gehäuses verteilt sind.

2. Gerät nach Anspruch 1, **dadurch gekennzeichnet, dass** die Struktur der dritten Schicht (42) in Form mehrerer konzentrischer Kavitäten (422) ausgeführt ist.

3. Gerät nach Anspruch 1, **dadurch gekennzeichnet, dass** die Struktur der dritten Schicht (42) mehrere parallele geradlinige Kavitäten (420) beinhaltet, die Gräben bilden, und eine Kammstruktur, die mehrere Zinken mit bestimmter Höhe und Breite aufweist.

4. Gerät nach Anspruch 1, **dadurch gekennzeichnet, dass** die Struktur der dritten Schicht (42) eine Spirale beinhaltet.

5. Gerät nach einem der Ansprüche 1 bis 4, **dadurch gekennzeichnet, dass** jede Kavität der Struktur durchgehend ist.

6. Gerät nach einem der Ansprüche 1 bis 5, **dadurch gekennzeichnet, dass** die zweite Schicht (41) aus einem Metall mit starker Wärmeleitfähigkeit wie Kupfer, Gold oder Aluminium gebildet ist.

7. Gerät nach einem der Ansprüche 1 bis 6, **dadurch gekennzeichnet, dass** die Wärmeleitpaste der ersten Schicht (40) und der dritten Schicht (42) eine Wärmeleitfähigkeit zwischen 1 und 12 W/m/K aufweist.

8. Gerät nach einem der Ansprüche 1 bis 7, **dadurch gekennzeichnet, dass** die erste Schicht (40) und die dritte Schicht (42) gefülltes Silikon beinhalten.

9. Gerät nach einem der Ansprüche 1 bis 8, **dadurch gekennzeichnet, dass** das Wärmeableitungs- und Stoßabsorptionsmodul (4) eine oder mehrere zusätzliche Schichten aus pyrolytischem Graphit beinhaltet, die unter der zweiten Schicht (41) und zwischen der dritten Schicht (42) und der Innenwand (10) des Gehäuses aufgebracht sind.

10. Verfahren zur Herstellung eines implantierbaren medizinischen Geräts wie in einem der Ansprüche 1 bis 9 definiert, **dadurch gekennzeichnet, dass** die Struktur der dritten Schicht (42) ausgeführt wird, indem Stanzelemente auf eine Schicht (50) aus Wärmeleitpaste gesetzt werden, die auf ein Substrat (51) aufgebracht ist, durch Formpressen oder Spritzgießen in einem chemisch löslichen oder thermisch verflüssigbaren Formwerkzeug (60).

**Claims**

1. Implantable medical device comprising:

   - an airtight housing (1) defining an internal space,
   - at least one electronic circuit board (2) housed in said internal space of the housing, said board supporting electronic components,
   - a heat sink and shock absorption module (4), housed in the internal space of the housing and arranged between said electronic circuit board (2) and an internal wall (10) of the housing, and

   **characterized in that** said heat sink and shock absorption module comprises:

   - a first layer (40) of thermal paste placed in the internal space of the housing and deposited on said electronic circuit board (2),
   - a second, metallic layer (41) with high thermal conductivity deposited on said first layer (40),
   - a third layer (42) of thermal paste deposited on said second layer (41) and arranged to bear against said internal wall (10) of the housing, said third layer (42) comprising a structure having several cavities distributed over the entire zone of contact of the third layer with the internal wall of the housing.

2. Device according to Claim 1, **characterized in that** the structure of the third layer (42) is produced in the form of several concentric cavities (422).

3. Device according to Claim 1, **characterized in that** the structure of the third layer (42) comprises several parallel rectilinear cavities (420) forming trenches and a comb structure having several teeth of determined height and width.

4. Device according to Claim 1, **characterized in that** the structure of the third layer (42) comprises a spiral.

5. Device according to one of Claims 1 to 4, **characterized in that** each cavity of the structure goes right through the structure.

6. Device according to one of Claims 1 to 5, **characterized in that** the second layer (41) is formed by a metal of high thermal conductivity such as copper, gold or aluminium.

7. Device according to one of Claims 1 to 6, **characterized in that** the thermal paste of the first layer (40) and of the third layer (42) has a thermal conductivity of between 1 and 12 W/m/K.

8. Device according to one of Claims 1 to 7, **charac-**

**terized in that** the first layer (40) and the third layer (42) comprise charged silicone.

9.  Device according to one of Claims 1 to 8, **characterized in that** the heat sink and shock absorption module (4) comprises one or more additional layers made of pyrolytic graphite, deposited under the second layer (41) and between the third layer (42) and the internal wall (10) of the housing.

10. Method for manufacturing an implantable medical device as defined in one of Claims 1 to 9, **characterized in that** the structure of the third layer (42) is produced by applying dies to a layer (50) of thermal paste deposited on a substrate (51), by compression moulding or by injection moulding in a chemically soluble or thermally liquefiable mould (60).

**Fig. 1**

**Fig. 2A**

**Fig. 2B**

**Fig. 2C**

**Fig. 2D**

*Fig. 3*

E1

50

51

E2

52

50

51

(X)

E3

52

50

51

E4

42

41

40

2

20

*Fig. 4*

E10

60

41

40

2

20

E20

60

61

41

40

2

E30

60

61

41

40

2

E40

42

41

40

2

## Fig. 5

E100

E200

E300

E400

*FIG. 6A*

*FIG. 6B*

*FIG. 6C*

**FIG. 7A**

**FIG. 7B**

**FIG. 7C**

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2006081361 A2

**Littérature non-brevet citée dans la description**

- **MESTAIS, C. S. ; CHARVET, G. ; SAUTER-STA-RACE, F. ; FOERSTER, M. ; RATEL, D. ; BENABID, A. L.** WIMAGINE: wireless 64-channel ECoG recording implant for long term clinical applications. *IEEE transactions on neural systems and rehabilitation engineering,* 2015, vol. 23 (1), 10-21